# EUROPEAN PATENT APPLICATION

(11) **EP 3 821 894 A1**
(43) Date of publication of application: **19.05.2021**
(21) Application number: 20212587.8
(22) Date of filing: 07.10.2011
(51) Int. Cl.: A61K 31/554

(54) **CONTROLLED-RELEASE FORMULATIONS OF QUETIAPINE**

(30) Priority: 08.10.2010 TR 201008261
(62) Divisional of application: 16170271.7
(71) Applicant: Sanovel Ilac Sanayi ve Ticaret A.S., 34460 Sariyer/Istanbul (TR)
(72) Inventor: Cifter, Ümit, 34398 Istanbul (TR); Türkyilmaz, Ali, 34398 Istanbul (TR); Yildirim, Ediz, 34398 Istanbul (TR); Erdem, Yelda, 34398 Istanbul (TR); Ergenekon, Ercan, 34398 Istanbul (TR); Öner, Levent, 34398 Istanbul (TR)
(74) Representative: Sonnenhauser, Thomas Martin

(57) **Abstract**

The present invention provides a pharmaceutical formulation comprising quetiapine or a pharmaceutically acceptable salt, solvate, or polymorph thereof, characterized in that said formulation comprise a non-gelling ethyl cellulose with an ethoxyl content of 48.0 - 51.5%.

## Description

### Field of Invention

The present invention relates to a novel pharmaceutical formulation comprising quetiapine or a pharmaceutically acceptable salt, solvate or hydrate thereof. The invention more particularly relates to a controlled-release formulation of quetiapine, uniformly dispersed in a matrix tablet and used as a single daily dose.

### Background of Invention

Quetiapine fumarate, a dibenzothiazepine derivative, is an atypical antipsychotic. It ameliorates the negative and positive symptoms of schizophrenia, without giving rise to extrapiramidal side effects. Similar to clozapine, quetiapine shows moderate dopamine D2-receptor antagonist and potent 5-HT2-receptor antagonist effects. The chemical designation of quetiapine fumarate is 2-[2-(4-dibenzo[b,f][1,4]thiazepine-11-yl-1-piperazinyl)ethoxy] ethanol fumarate, with the following chemical structure of Formula I.

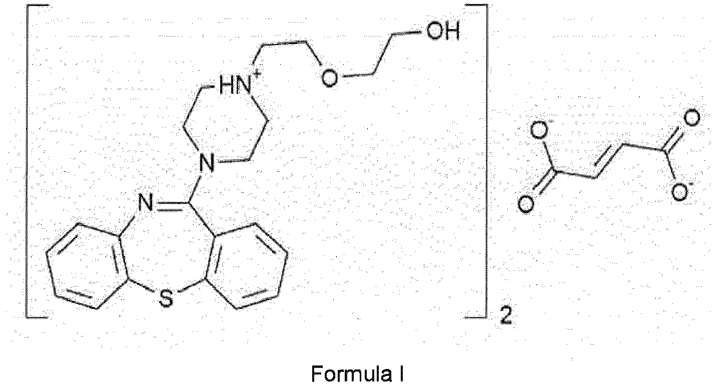

Extended release tablets of quetiapine is marketed under the name Seroquel XR® and is administered orally once in a day. These tablets comprise 50 mg, 150 mg, 200 mg, 300 mg or 400 mg quetiapine fumarate as an active agent.

The quetiapine molecule was disclosed in the applications EP0240228 and US4879288. EP282236 discloses a process for the preparation of a quetiapine molecule.

The patent EP2153834 discloses an extended release multiparticulate formulation comprising quetiapine or a pharmaceutical acceptable salt thereof and an excipient and hydrophobic release controlling agent.

The patent US20080287418 discloses an extended release formulation comprising 9.6 - 10.4% quetiapine, 30% hydroxypropyl methyl cellulose, and 7.2% sodium citrate dihydrate.

The patent WO2010012490 discloses a controlled release formulation comprising quetiapine and a non-gelling carrageenan.

The patent WO9745124 discloses a formulation comprising gelling agent, quetiapine, and one or more excipients. There are mentioned on various polymers in that patent for use as a gelling agent. Hydroxypropyl methyl cellulose, hydroxypropyl cellulose, hydroxymethyl cellulose, hydroxyethyl cellulose, carbomer, sodium carboxymethyl cellulose, polyvinylpyrrolidone, ethyl cellulose, methyl cellulose, carboxymethyl cellulose are specified in that document as gelling agents.

It is a desired feature to obtain an active pharmaceutical agent in a modified-release form in the treatment of many diseases. What is desired with a modified release is to reach desired plasma levels of an active agent of interest throughout a determined period of time, and to provide the drug release at a uniform and constant rate. Thus, the drug administration frequency can be lowered.

There are various difficulties associated with developing a controlled-release formulation. One of the problems encountered is dose damping, by which a drug obtained is released too rapidly. Obtaining intended solubility profiles is also difficult, i.e. it is difficult to control the release rate. For this reason, there may occur variations in the plasma concentrations of active agents, which may lead to toxicity. In addition, there are also daily alterations possible for the active agent in plasma.

There are various formulations available, which have been developed with quetiapine fumarate. There are various problems, however, associated with quetiapine fumarate formulations.

Quetiapine fumarate has a low dissolution rate. Producing controlled-release tablets of quetiapine fumarate and similar active agents having low solubility rates, and providing desired release profiles in these tablets are quite difficult. Generating release amounts at desired intervals bears vital importance with respect to patients. Obtaining a desired release profile depends on the convenience of the excipients to be selected.

Quetiapine fumarate is also a low-density substance. Therefore the tablet production process of quetiapine fumarate is quite difficult. Tablets produced are prone to erosion and disintegration.

Quetiapine fumarate is to be stored below temperatures of 30°C under normal conditions. Stability-related problems are encountered in temperatures exceeding this point. Failing to prepare a formulation with convenient excipients leads to stability problems.

Accordingly, there is a need towards modified-release formulations of quetiapine or a pharmaceutically-acceptable salt thereof, which would overcome the problems referred to hereinabove, and would provide drug plasma concentrations equivalent or better than those of the currently-used immediate-release tablet formulations. The formulation according to the present invention provides a novel modified-release formulation, not disclosed in the relevant art yet.

The modified-release formulation according to the present invention both provides plasma levels comparable to the immediate-release form thereof, and gives an advantageous feature of administering the active drug less frequently, e.g. once or twice a day.

Due to the reasons specified above, there is a need towards a stable pharmaceutical formulation of quetiapine or pharmaceutically acceptable salts, solvates, or hydrates thereof, providing a proper content uniformity and a desired release profile.

### Object and Brief Description of Invention

The present invention relates to a controlled-release formulation of quetiapine, eliminating all aforesaid problems and brining additional advantages to the relevant prior art.

Accordingly, the main object of the present invention is to obtain a controlled-release formulation comprising quetiapine, which is stable and provides a desired release profile.

Another object of the present invention is to obtain a controlled-release formulation with a desired dissolution rate thanks to employing proper excipients.

A further object of the present invention is to ensure a uniform distribution of active agent and excipients in the formulation, so that a tablet form thereof will not be prone to erosion.

A controlled-release pharmaceutical formulation comprising quetiapine or a pharmaceutically acceptable salt, solvate, or polymorph thereof has been developed to carry out all objects, referred to above and to emerge from the following detailed description.

According to a preferred embodiment of the present invention, said novelty is carried out with non-gelling ethyl cellulose with a ethoxyl content in between 48.0 to 52.5%. Thanks to the present invention developed, a stable controlled-release formulation is surprisingly obtained thanks to making use of ethyl cellulose with an ethoxyl content of 48 - 52.5% in which quetiapine is uniformly distributed, thereby solving said problems and allowing to achieve the desired release profile. Thanks to the formulation developed, the risk of dose damping is eliminated and a controlled-release formulation is obtained by using non-gelling ethyl cellulose.

According to a preferred embodiment of the present invention, as much as 35%, preferably as much as 30% of quetiapine or a pharmaceutically acceptable salt thereof is released in 2 hours; 30 to 65%, preferably 35 to 55% of quetiapine or a pharmaceutically acceptable salt thereof is released in 4 hours; and a minimum of 80% of quetiapine or a pharmaceutically acceptable salt thereof is released in 16 hours.

According to a preferred embodiment of the present invention, the weight ratio of quetiapine to ethyl cellulose is between 0.1 to 10, preferably 1 to 4, and more preferably 1.5 to 3.

According to a preferred embodiment of the present invention, the proportion by weight of ethyl cellulose to the total weight of formulation is between 10 to 65%, preferably 15 to 50%, and more preferably between 20 to 40%.

According to another preferred embodiment of the present invention, said quetiapine is in the form of a fumarate salt thereof.

According to another preferred embodiment of the present invention, excipients used comprise at least one or a mixture of fillers, buffering agents, glidants, lubricants.

According to a preferred embodiment of the present invention, said filler is at least one of microcrystalline cellulose and lactose monohydrate, or a properly-proportioned mixture thereof.

According to a preferred embodiment of the present invention, said buffering agent is sodium citrate dihydrate. With using sodium citrate dihydrate in the formulation, the desired release profile can be obtained independent from the pH of the medium.

According to another preferred embodiment of the present invention, the proportion by weight of ethyl cellulose to sodium citrate dihydrate is between 0.2 to 60, preferably 1 to 50, and more preferably between 2 to 40.

According to a preferred embodiment of the present invention, said glidant is colloidal silicon dioxide.

According to a preferred embodiment of the present invention, magnesium stearate is used as a lubricant.

According to a preferred embodiment of the present invention, said formulation coating does not comprise ethyl cellulose with an ethoxyl content of 43- 50 %.

According to a preferred embodiment of the present invention, said tablet is uncoted tablet.

A further preferred embodiment according to the present invention provides a method for preparing a pharmaceutical formulation, this method comprising the steps of
a. mixing quetiapine, filler and controlled-release agent together,
b. forming wet granulation with an alcohol-water mixture,
c. wet milling and drying,
d. sieving the granules,
e. adding lubricant-glidant into the sieved powder mixture obtained with granular structure and mixing the latter,
f. compressing into tables, and
g. coating the tablets.

According to another preferred embodiment of to the present invention, said pharmaceutical formulation is consisting of
a. core
   a. quetiapine fumarate at 33 to 70% by weight
   b. ethyl cellulose at 10 to 65% by weight
   c. sodium citrate dihydrate at 2 to 30% by weight
   d. lactose monohydrate at 3.0 to 50% by weight
   e. microcrystalline cellulose at 4.0 to 60% by weight
   f. colloidal silicone dioxide at 0.2 to 5.0% by weight
   g. magnesium stearate at 0.1 to 5.0% by weight
b. coating
   a. hydroxymethylpropyl cellulose or polyvinyl alcohol at 1.0 to 5.0% by weight.

### Detailed Description of Invention

### Example

| **Content** | **Amount (%) (w/w)** |
|---|---|
| quetiapine fumarate | 51.2 |
| ethyl cellulose (ethoxyl content 48 - 52.5%) | 22.0 |
| sodium citrate dihydrate | 3.0 |
| lactose monohydrate | 4.6 |
| microcrystalline cellulose | 16.2 |
| colloidal silicone dioxide | 0.6 |
| magnesium stearate | 2.4 |

Ethyl celluloses, commercially available under the trademark Aqualon®, used in the present invention has four different types according their ethoxyl content.

| **Type** | **Ethoxyl content (%)** |
|---|---|
| K type | 45 - 47,2 |
| N type | 48-49,5 |
| T type | 49,6 - 51,5 |
| X type | 50,5 - 52,5 |

(see the 2002-document specifying physical and chemical specifications of the product Aqualon ® of the company Herkules, p. 3.)

Ehtyl celluloses with type K, N, T, X, as indicated in the table, have different ethoxyl contents and show characteristic features in line with content rates. The type K, among these 4 types of ethyl cellulose gelates in organic solvents (toluene, xylene, butyl acetate, carbon tetrachloride), but the types N, T, and X does not gelate. With the invention made, at least one or a properly-proportioned mixture of the type N, T, and X ethyl celluloses are used.

The N type and/or T type ethyl cellulose used in the formulation does not dissolve in water and becomes not gelated. Specifically-selected excipients are used together with the N type and/or T type ethyl cellulose in the formulation. Sodium citrate dihydrate, among these excipients, generates a proper pH medium around the ethyl cellulose matrix and facilitates its solubility. Thanks to this feature, the release profile of the formulation is brought to the desired level independently from the pH of the medium.

Thanks to the present invention developed, a stable controlled-release formulation is surprisingly obtained thanks to making use of ethyl cellulose with an ethoxyl content of 48 - 52.5% in which quetiapine is uniformly distributed, thereby solving said problems and allowing to achieve the desired release profile. Thanks to the formulation developed, the risk of dose damping is eliminated and a controlled-release formulation is obtained by using non-gelling ethyl cellulose. Preferably core of said formulation comprise a non-gelling ethyl cellulose with an ethoxyl content of 48 - 51,5 %. More preferably core of said formulation comprise a non-gelling ethyl cellulose with an ethoxyl content of 48 - 49,5 %.

The formulation is made as follows. Quetiapine, filling agent, and controlled-release agent are mixed together. Then this mixture is subjected to a wet milling process, together with an alcohol-water mixture. Then, the granules obtained by wet milling and then dried are sieved. Thereafter, lubricant-glidant is added to the sieved powder mixture with a granular form and the resulting mixture is mixed again. Finally, the mixture obtained is compressed into tablets and the tablets obtained are coated.

Another preferred application in relation to the formulation is that they are preferred in dependence on the viscosity ranges of ethyl cellulose used. The N type ethyl cellulose selected for use in one embodiment has the following viscosity ranges.

| **Type** | **Viscosity range (mPa.s)** |
|---|---|
| N7 Pharm | 6 - 8 |
| N10 Pharm | 8 - 11 |
| N14 Pharm | 12 - 16 |
| N22 Pharm | 18 - 24 |
| N50 Pharm | 40 - 52 |
| N100 Pharm | 80 - 105 |
| T 10 Pharm | 8 - 11 |
| T 50 Pharm | 40 - 52 |

In the subject formulations are employed the N type ethyl cellulose with a 18-24 mPa.s viscosity range, among the specified N type products with a viscosity range of 5.6 to 105 mPa.s, and the T type ethyl cellulose with a 8-11 mPa.s viscosity range among the specified T type products.

It is further possible to use the following additional excipients in the formulation.

Suitable binders include, but are not restricted to at least one or a mixture of polyvinylpyrrolidone, gelatin, sugars, glucose, natural gums, gums, synthetic celluloses, polymethacrylate, hydroxypropyl methyl cellulose, hydroxypropyl cellulose, carboxymethyl cellulose, methyl cellulose, and other cellulose derivatives.

Suitable glidants include, but are not restricted to at least one or a mixture of colloidal silicone dioxide, talk, aluminum silicate.

Suitable lubricants include, but are not restricted to at least one or a mixture of sodium stearil fumarate, magnesium stearate, polyethylene glycol, stearic acid, metal stearates, boric acid, sodium chloride benzoate and acetate, sodium or magnesium lauryl sulfate.

Suitable colorants include, but are not restricted to at least one or a mixture of food, drug, and cosmetic (FD&C) dyes (FD&C blue, FD&C green, FD&C red, FD&C yellow, FD&C lake), ponceau, indigo Drug & cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow.

Treatment of diseases, such as bipolar disease, obsessive-compulsive disorder and schizophrenia, mania, depression, dementia, agitation disorders can be provided with the formulation made according to the present invention.

The protection scope of the present invention is set forth in the annexed claims and cannot be restricted to the illustrative disclosures given above, under the detailed description. Any alternative embodiments to be produced by persons skilled in the art according to the basic principles, which are under the protection scope as set forth in the claims, shall be an infringement of the present invention.

Further embodiments of the application are as follows:
1. A pharmaceutical formulation comprising quetiapine or a pharmaceutically acceptable salt, solvate, or polymorph thereof, characterized in that core of said formulation comprise a non-gelling ethyl cellulose with an ethoxyl content of 48 - 51,5 %.
2. The controlled-release formulation according to embodiment 1, wherein core of said formulation comprise a non-gelling ethyl cellulose with an ethoxyl content of 48 - 49,5 %.
3. The controlled-release formulation according to any of the preceding embodiments, wherein the weight ratio of quetiapine to ethyl cellulose is between 0.1 to 10, preferably 1 to 4, and more preferably 1.5 to 3.
**4.** The controlled-release formulation according to any of the preceding embodiments, wherein the proportion by weight of ethyl cellulose to the total weight of formulation is between 10 to 65%, preferably 15 to 50%, and more preferably between 20 to 40%.
5. The pharmaceutical formulation according to any of the preceding embodiments, wherein said quetiapine is in the form of its fumarate salt.
6. The controlled-release formulation according to any of the preceding embodiments, further containing excipients, comprising at least one or a mixture of fillers, buffering agents, glidants, lubricants.
7. The pharmaceutical formulation according to any of the preceding embodiments, wherein said filler is at least one of microcrystalline cellulose and lactose monohydrate, or a properly-proportioned mixture thereof.
8. The pharmaceutical formulation according to any of the preceding embodiments, wherein said buffering agent is sodium citrate dihydrate.
9. The controlled-release formulation according to any of the preceding embodiments, wherein the weight ratio of ethyl cellulose to sodium citrate dihydrate is between 0.2 to 60, preferably 1 to 50, and more preferably between 2 to 40.
10. The pharmaceutical formulation according to any of the preceding embodiments, wherein said glidant is colloidal silicone dioxide.
11. The pharmaceutical formulation according to any of the preceding embodiments, wherein said lubricant is magnesium stearate.
12. A method for preparing a pharmaceutical formulation according to any of the preceding embodiments, comprising the steps of
   a. mixing quetiapine, filler and controlled-release agent together,
   b. forming wet granulation with an alcohol-water mixture,
   c. wet milling and drying,
   d. sieving the granules,
   e. adding lubricant-glidant into the sieved powder mixture obtained with granular structure and mixing the latter,
   f. compressing into tables, and
   g. coating the tablets.
13. The pharmaceutical formulation according to any of the preceding embodiments, consisting of
   a. core
      a. quetiapine fumarate at 33 to 70% by weight
      b. ethyl cellulose at 10 to 65% by weight
      c. sodium citrate dihydrate at 2 to 30% by weight
      d. lactose monohydrate at 3.0 to 50% by weight
      e. microcrystalline cellulose at 4.0 to 60% by weight
      f. colloidal silicone dioxide at 0.2 to 5.0% by weight
      g. magnesium stearate at 0.1 to 5.0% by weight
   b. coating
      a. hydroxymethylpropyl cellulose or polyvinyl alcohol at 1.0 to 5.0% by weight.
14. A pharmaceutical formulation according to any of the preceding embodiments for preventing or treating bipolar disease, obsessive-compulsive disorder and schizophrenia, mania, depression, dementia, agitation disorders in mammalians and particularly in humans.

## Claims

1. A pharmaceutical formulation in the form of a tablet comprising a core and an optional coating, the core comprising quetiapine or a pharmaceutically acceptable salt, solvate, or polymorph thereof, **characterized in that** the core of said formulation comprises a non-gelling ethyl cellulose with an ethoxyl content of 48 - 51,5% and a viscosity of 40- 52 mPa.

2. The controlled-release formulation according to claim 1, wherein the weight ratio of quetiapine to ethyl cellulose is between 0.1 to 10, preferably 1 to 4, and more preferably 1.5 to 3.

3. The controlled-release formulation according to any of the preceding claims, wherein the proportion by weight of ethyl cellulose to the total weight of formulation is between 10 to 65%, preferably 15 to 50%, and more preferably between 20 to 40%.

4. The pharmaceutical formulation according to any of the preceding claims, wherein said quetiapine is in the form of its fumarate salt.

5. The controlled-release formulation according to any of the preceding claims, further containing excipients, comprising at least one or a mixture of fillers, buffering agents, glidants, lubricants.

6. The pharmaceutical formulation according to any of the preceding claims, wherein said filler is at least one of microcrystalline cellulose and lactose monohydrate, or a properly-proportioned mixture thereof.

7. The pharmaceutical formulation according to any of the preceding claims, wherein said buffering agent is sodium citrate dihydrate.

8. The controlled-release formulation according to any of the preceding claims, wherein the weight ratio of ethyl cellulose to sodium citrate dihydrate is between 0.2 to 60, preferably 1 to 50, and more preferably between 2 to 40.

9. The pharmaceutical formulation according to any of the preceding claims, wherein said glidant is colloidal silicone dioxide.

10. The pharmaceutical formulation according to any of the preceding claims, wherein said lubricant is magnesium stearate.

11. A method for preparing a pharmaceutical formulation according to any of the preceding claims, comprising the steps of
a) mixing quetiapine, filler and controlled-release agent together,
b) forming wet granulation with an alcohol-water mixture,
c) cwet milling and drying,
d) sieving the granules,
e) adding lubricant-glidant into the sieved powder mixture obtained with granular
f) structure and mixing the latter,
g) compressing into tablets, and
h) coating the tablets.

12. The pharmaceutical formulation according to any of claim 1-10, consisting of
A. a core, comprising
a) quetiapine fumarate at 33 to 70% by weight
b) ethyl cellulose at 10 to 65% by weight
c) sodium citrate dihydrate at 2 to 30% by weight
d) lactose monohydrate at 3.0 to 50% by weight
e) microcrystalline cellulose at 4.0 to 60% by weight
f) colloidal silicone dioxide at 0.2 to 5.0% by weight
g) magnesium stearate at 0.1 to 5.0% by weight; and
B. a coating, comprising
a) hydroxymethylpropyl cellulose or polyvinyl alcohol at 1.0 to 5.0% by weight.

13. A pharmaceutical formulation according to any of claims 1-10 or 12 for use in preventing or treating bipolar disease, obsessive-compulsive disorder and schizophrenia, mania, depression, dementia, agitation disorders in mammalians and particularly in humans.
